(19) Europäisches Patentamt
European Patent Office
Office européen des brevets

(11) **EP 4 659 769 A1**

(12) **EUROPEAN PATENT APPLICATION**

(43) Date of publication:
**10.12.2025 Bulletin 2025/50**

(21) Application number: **24198481.4**

(22) Date of filing: **04.09.2024**

(51) International Patent Classification (IPC):
*A61L 15/24* [(2006.01)]   *A61L 15/34* [(2006.01)]
*A61L 15/60* [(2006.01)]   *B01J 20/24* [(2006.01)]
*C08J 3/12* [(2006.01)]   *C08J 3/24* [(2006.01)]
*B01J 20/30* [(2006.01)]

(52) Cooperative Patent Classification (CPC):
**C08J 3/245; A61L 15/60; B01J 20/24;
B01J 20/3242; C08J 3/126;** C08J 2301/02;
C08J 2333/02

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **07.06.2024 TW 113121387**

(71) Applicant: **Formosa Plastics Corporation
Kaohsiung City (TW)**

(72) Inventors:
• **CIOU, Yan-Syun
Kaohsiung City (TW)**

• **CHEN, Feng-Yi
Kaohsiung City (TW)**
• **CHEN, Zhong-Yi
Kaohsiung City (TW)**

(74) Representative: **Lang, Christian
LangPatent Anwaltskanzlei IP Law Firm
Ingolstädter Straße 5
80807 München (DE)**

Remarks:
Amended claims in accordance with Rule 137(2)
EPC.

(54) **SUPERABSORBENT POLYMER AND METHOD OF MAKING THE SAME**

(57)    The present invention relates to a superabsorbent polymer and a method of making the same. The superabsorbent polymer includes a cross-linking core structure and a shell layer. The shell layer encloses the cross-linking core structure completely, and the shell layer includes microcrystalline cellulose. The superabsorbent polymer has not only good absorption properties but also excellent absorption rate as well as permeability, and can be applied to thinner hygiene products.

100

FIG. 1

EP 4 659 769 A1

## Description

### BACKGROUND

Field of Invention

The present invention relates to a superabsorbent polymer. More particularly, the present application provides a superabsorbent polymer obtained by performing a surface-treating step with a surface-treating agent including microcrystalline cellulose and a method of making the same.

Description of Related Art

A superabsorbent polymer is a polymer material that can absorb and retain 50 times to 500 times or more its original mass in a liquid. With excellent absorption properties, the superabsorbent polymer can be widely applied in personal hygiene products (e.g., baby diapers, sanitary products, disposable wipes and/or adult diapers). Moreover, the superabsorbent polymer can also be applied in agriculture and horticulture (e.g., water retaining agents), building material (e.g., as anti-dew condensation agents), oil industry (e.g., as water remover), cable manufacturing industry (e.g., waterproof covering agent), pharmaceutical industry (e.g., wound dressing) and entertainment industry (e.g., expansion toy, artificial snow), etc.

In tends to meet the market demand for thinner hygiene products, as exemplified by diapers, it is required to reduce the usage amount of hydrophilic fibers (i.e. wood pulp or materials with low bulk density) and increase the usage amount of the superabsorbent polymer (i.e. materials with high bulk density and water absorption amount). However, in fact, as the usage amount of the superabsorbent polymer of the diaper increases, but the usage amount of the hydrophilic fibers decreases, the surface of the superabsorbent polymer gets wet and swells easily due to moisture in the liquid, resulting in the defect of gel blocking. In the gel blocking situation, liquid can move through the swollen superabsorbent polymer only by diffusion, leading to the reduced absorption properties of the diapers.

To prevent gel blocking, the superabsorbent polymer can be modified by the conventional methods. In U.S. Pat. No. US 10,335,768B, the surface of the superabsorbent polymer was subjected to the polymerization reaction with UV light irradiation, so as to trigger a second surface-treating (crosslinking) step on the surface of the superabsorbent polymer. In Japan patent publication number JP 2001-523289A, the superabsorbent polymer was mixed with polyvalent metal salt (e.g., aluminum sulfate), followed by a heating reaction. Japan patent JP 2509087 disclosed to mix the superabsorbent polymer (with a particle size of 5 $\mu$m to 500 $\mu$m) with polyvalent metal salt and then perform a heating reaction. In China patent publication number CN 1747751A, WIPO patent publication number WO 2004/113452A, and U.S. patent publication number US 2017/0050170A, the superabsorbent polymer was mixed with water-soluble polyvalent metal powders and adhesive. In U.S. patent publication number US 5985944A, an azo compound containing amine groups as a foaming agent was added into the aqueous solution of acid group-containing monomers. In Japan Pat. No. JP 6532894B, a surfactant (e.g. polyoxyethylene sorbitan fatty acid ester) was added into the aqueous solution of acid group-containing monomers, so as to decrease a solubility of gas dissolved in the monomer aqueous solution. In U.S. patent publication number US 2005/0245684A, a nitrogen-containing polymer with nitrogen atoms that could be protonated (such as N-vinylcarboxamide), or its solution was added to the superabsorbent polymer. In U.S. Pat. No. US 7,777,093B, the surface of the superabsorbent polymer was coated with thermoplastic polymers (such as polyurethane, polyester, or the like). In WIPO patent publication number WO 2006/082189A, a surface-treating step was performed on the surface of the superabsorbent polymer with polyvinylamine or polyallylamine.

However, the additional adding of raw materials of the superabsorbent polymer not only raises concerns about the safety of the raw materials but also increases the overall cost, further lowering other physical properties of the superabsorbent polymer.

Accordingly, there is an urgent need to provide a superabsorbent polymer and a method of making the same to improve the abovementioned problems.

### SUMMARY

Therefore, an aspect of the present application is to provide a superabsorbent polymer. The superabsorbent polymer includes a cross-linking core structure and a shell layer, in which the shell layer includes microcrystalline cellulose. The superabsorbent polymer has good absorption properties and excellent absorption rate and permeability.

Another aspect of the present application is to provide a method of making a superabsorbent polymer. In the method, a surface-treating step is performed with a surface-treating agent including microcrystalline cellulose, so the formed shell layer includes microcrystalline cellulose, thereby increasing the absorption rate and permeability of the obtained superabsorbent polymer.

Another aspect of the present invention is to provide a superabsorbent polymer. The superabsorbent polymer includes a cross-linking core structure and a shell layer, in which the cross-linking core structure is obtained by subjecting free radical polymerization step on an aqueous solution of acid group-containing monomers, a crosslinking agent, and a polymerization initiator. The shell layer encloses the cross-linking core structure completely, in which the shell layer is formed by performing a surface-treating step on a cross-linking core structure with a surface-treating agent. The surface-treating agent including microcrystalline cellulose. Based on the cross-linking core structure is 100 weight percent, the usage amount of the microcrystalline cellulose is 0.1 weight percent to 5.0 weight percent.

According to some embodiments of the present invention, based on a total solid content of the superabsorbent polymer as 100 weight percent, the usage amount of the crosslinking agent is 0.001 weight percent to 5 weight percent.

According to some embodiments of the present invention, the polymerization initiator includes a thermal decomposition initiator and/or a redox initiator.

According to some embodiments of the present invention, based on the total solid content of the superabsorbent polymer as 100 weight percent, the usage amount of the surface-treating agent is 0.001 weight percent to 10 weight percent.

According to some embodiments of the present invention, a particle size of the microcrystalline cellulose is 20 $\mu$m to 100 $\mu$m.

According to some embodiments of the present invention, a bulk density of the microcrystalline cellulose is 0.25 to 0.35.

According to some embodiments of the present invention, a moisture content of the microcrystalline cellulose is smaller than or equal to 7%.

According to some embodiments of the present invention, a degree of polymerization of the microcrystalline cellulose is smaller than or equal to 350.

Another aspect of the present invention is to provide a method of making the superabsorbent polymer. First, a free radical polymerization step is performed on the superabsorbent polymer composition, so as to form a cross-linking core structure. The superabsorbent polymer composition includes an aqueous solution of acid group-containing monomers, a crosslinking agent and a polymerization initiator. The polymerization initiator includes a thermal decomposition initiator and/or a redox initiator.

Then, a surface-treating step is performed on the aforementioned cross-linking core structure with a surface-treating agent, so as to form a cross-linking core structure on a shell layer, thereby obtaining a superabsorbent polymer. The aforementioned shell layer encloses the cross-linking core structure completely. The surface-treating agent includes microcrystalline cellulose. Based on the cross-linking core structure of the superabsorbent polymer is 100 weight percent, the usage amount of the microcrystalline cellulose is 0.1 weight percent to 5.0 weight percent.

According to some embodiments of the present invention, the particle size of the microcrystalline cellulose is 20 $\mu$m to 100 $\mu$m.

According to some embodiments of the present invention, a bulk density of the microcrystalline cellulose is 0.25 to 0.35.

According to some embodiments of the present invention, a moisture content of the microcrystalline cellulose is smaller than or equal to 7%.

According to some embodiments of the present invention, the degree of polymerization of the microcrystalline cellulose is smaller than or equal to 350.

According to some embodiments of the present invention, a neutralizing step is selectively performed on the aqueous solution of acid group-containing monomers before the free radical polymerization step.

According to some embodiments of the present invention, a drying step and/or a pulverizing step on the cross-linking core structure is selectively performed before the surface-treating step, so as to obtained a powder of the cross-linking core structure.

In the superabsorbent polymer and the method of making the same of the present invention, the superabsorbent polymer includes a cross-linking core structure as well as a shell layer including microcrystalline cellulose, and therefore the superabsorbent polymer has not only good absorption properties but also excellent absorption rate and permeability, thereby being able to be applied to thinner hygiene products to improve defects of the conventional superabsorbent polymer such as gel blocking.

## BRIEF DESCRIPTION OF THE DRAWINGS

The application can be more fully understood by reading the following detailed description of the embodiment, with reference made to the accompanying drawings as follows:

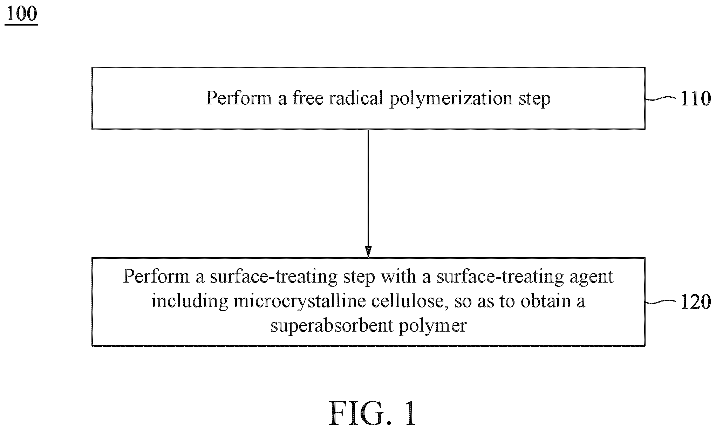

FIG. 1 illustrates a flow chart of a method for making a superabsorbent polymer according to some embodiments of the present invention.

**DETAILED DESCRIPTION**

As mentioned above, the present invention provides a superabsorbent polymer and a method of making the same. The superabsorbent polymer includes a cross-linking core structure and a shell layer, in which the shell layer includes microcrystalline cellulose, such that the superabsorbent polymer has not only good absorption properties, but also excellent absorption rate and permeability. Therefore, defects of the conventional superabsorbent polymer such as gel blocking can be improved.

The term "absorption properties" herein is referred to the capacity of the superabsorbent polymer to absorb liquid. In some specific examples, the liquid can include but not limited to pure water, saline, urine, menstrual blood, soil water, or other water solution applied in the environment. It is noted that salt may affect the absorbent properties of the superabsorbent polymer. Therefore, the weight of the saline or urine that a superabsorbent polymer can absorb is smaller than that of pure water. To simulate the real situation, the absorbent properties of the superabsorbent polymer can be evaluated by using saline or synthetic urine.

In some embodiments, the absorption properties can be evaluated by absorption rate (also referred to as free swell capacity, FSC) that is ratio of the maximum weight of the liquid absorbed by a superabsorbent polymer with no external force exerted to the dry weight of the superabsorbent polymer that has not absorbed the liquid.

In some embodiments, the absorption properties can be evaluated by absorption against pressure (AAP). AAP represents ratio of a weight of liquid absorbed and retained in the superabsorbent polymer under a certain pressure for a considerable period to a dry weight of the superabsorbent polymer that has not absorbed the liquid. AAP of not less than 15 g/g, e.g., 20 g/g to 30 g/g, represents excellent absorption properties of the superabsorbent polymer.

The term "absorption rate" herein can be evaluated by the time that a unit weight of superabsorbent polymer needs to absorb a unit weight of liquid. The shorter the time is, the faster the absorption rate is. In some embodiments, the absorption rate can be evaluated by T20 obtained by the dynamic effective permeability and the absorption kinetics testing method (K(t) testing method) described hereafter. T20 represents the time for a dry superabsorbent polymer to absorb 20 times its own weight in saline. The smaller the T20 value is, the bigger the absorption rate of the superabsorbent polymer is.

In some embodiments, permeability can be evaluated by urine permeability measurement (UPM) value obtained by a urine permeability measurement described herein. UPM represents the flow resistance of a preswollen layer of the superabsorbent polymer, i.e., the flow resistance when the absorption of the superabsorbent polymer is in equilibrium. If a superabsorbent polymer has a high UPM value, it means that the superabsorbent polymer has permeability to liquid when the superabsorbent polymer is wet and swells and therefore can show excellent absorption properties on surges followed by the first surge.

In some embodiments, permeability can be evaluated by gel bed permeability (GBP) obtained by the FSGBP measuring method described hereafter. The GBP represents the permeability of the superabsorbent polymer to a liquid under the condition of free swelling. The term "free swelling" herein represents that the superabsorbent polymer is allowed to swell without a swelling restraint loading, i.e., the swelling pressure is 0 psi.

As mentioned above, the cross-linking core structure and the shell layer of the superabsorbent polymer of the present invention can be obtained by the following method. In the method, the surface-treating agent including microcrystalline cellulose is used to enclose the surface of the superabsorbent polymer, thereby enhancing the absorption rate and permeability of the obtained superabsorbent polymer while keeping the absorption properties of the superabsorbent polymer at the same time. If the superabsorbent polymer of the present invention is applied in thinner hygiene products (i.e., diapers, sanitary products, and pads), the defects of gel blocking of the conventional superabsorbent polymer can be effectively improved, thereby keeping the absorption properties of the superabsorbent polymer.

In the present invention, a free radical polymerization step is formed on a superabsorbent polymer composition at first, so as to form the hydrogels of the cross-linking core structures which are further cut into small hydrogels. Next, a drying step and a pulverizing step are performed on the small hydrogels to obtain powders of the cross-linking core structures. Hereafter, the surface-treating agent including the microcrystalline cellulose is used to perform a surface-treating step on the cross-linking core structures, so as to form a shell layer including the microcrystalline cellulose, thereby obtaining the superabsorbent polymer of the present invention.

It is noted that by forming the shell layer including the microcrystalline cellulose, the gap between particles of the superabsorbent polymer can be increased, and thus can enhance the fluidity of the liquid, thereby solving the conventional defects of gel blocking, but the superabsorbent polymer still keeps good absorption properties.

The method of making the superabsorbent polymer of the present invention includes at least: (a) performing a free radical polymerization step on an aqueous solution of acid group-containing monomers, so as to form a cross-linking core structure; (b) performing a surface-treating step on the cross-linking core structure with the surface-treating agent including the microcrystalline cellulose, so as to form the shell layer on the cross-linking core structure of the superabsorbent polymer. The shell layer encloses the cross-linking core structure completely and includes the microcrystalline cellulose. Please refer to FIG. 1, which illustrates a flow chart of a method 100 for making a superabsorbent polymer according to some embodiments of the present invention. In method 100, a free radical polymerization step is performed on the superabsorbent polymer composition to form a cross-linking core structure, as shown in operation 110. The superabsorbent polymer composition includes an aqueous solution of acid group-containing monomers, a crosslinking agent, and a polymerization initiator.

The aqueous solution of acid group-containing monomers is obtained by dissolving the acid group-containing monomers in water. In some embodiments, the acid group-containing monomers can include but not limited to acrylic acid, methacrylic acid, 2-propenylamine-2-methylpropanesulfonic acid, maleic acid, fumaric acid, maleic anhydride and/or fumaric anhydride. The aforementioned monomers can be used alone or be used with a combination of multi-monomers.

In some embodiments, other hydrophilic monomers having unsaturated double bonds can be selectively added depending on needs. In some specific examples, the other hydrophilic monomers having unsaturated double bonds can be acrylamide, methacrylamide, 2-carboxyethyl acrylate, 2-carboxyethyl methacrylate, methyl acrylate, ethyl acrylate, dimethylaminopropyl acrylamide, allylacrylamide trimethylamine chloride, or any mixture of other hydrophilic monomers mentioned above. There are no specific limitations to an amount of the other hydrophilic monomers having unsaturated double bonds as long as the physical properties (e.g., absorption properties, absorption rate, and permeability) of the superabsorbent polymer remains.

In some embodiments, the aqueous solution of acid group-containing monomers can selectively be subjected to a neutralizing step, so that the obtained superabsorbent polymer can be neutral or slightly acidic. The neutralizing agent used for the neutralizing step can be a basic compound which can include but not limited to sodium hydroxide, potassium hydroxide, sodium carbonate, potassium carbonate, sodium bicarbonate, potassium bicarbonate, an ammonia compound, other proper basic compounds, or any combination of the abovementioned basic compound. By using the abovementioned basic compound, carboxylic acid groups of the acid group-containing monomers can be partially neutralized to salts, e.g., sodium salts, potassium salts, or ammonium salts.

In some embodiments, a neutralization concentration of the aqueous solution of acid group-containing monomers can be 45 mole percent to 85 mole percent and preferably 50 mole percent to 75 mole percent. If the neutralization concentration of the aqueous solution of acid group-containing monomers is in the aforementioned range, the obtained superabsorbent polymer can be neutral or slightly acidic to prevent the human body from damage when contacting the skin. In some embodiments, the pH value of the aqueous solution of acid group-containing monomers can be not smaller than 5.5, for example, so that the obtained cross-linking core structure has fewer residual monomers, therefore being helpful to enhance the physical properties of the superabsorbent polymer.

In some embodiments, the time of the neutralizing step can be 1 hour to 2 hours, so as to control the neutralization concentration of the aqueous solution of acid group-containing monomers. In some embodiments, the temperature of the neutralizing step can be performed at room temperature (15°C to 40°C). In some embodiments, as the neutralizing step is performed, the neutralizing agent is slowly dropped into the aqueous solution of acid group-containing monomers, so that the temperature of the aqueous solution of acid group-containing monomers is controlled in the aforementioned range. In some specific examples, the dropping ratio of the neutralizing agent and the aqueous solution of acid group-containing monomers can be 0.85 to 0.95, for example.

As the free radical polymerization step is performed, the concentration of the aqueous solution of acid group-containing monomers is not specifically limited, and the concentration can preferably be 20 weight percent to 55 weight percent and preferably 30 weight percent to 45 weight percent. If the concentration of the aqueous solution of acid group-containing monomers is in the range, the cross-linking core structure formed by polymerization can have proper hardness and viscosity, thereby having better processing properties and fewer residual monomers in the cross-linking core structure, so that the obtained superabsorbent polymer can have better physical properties.

In some embodiments, the aqueous solution of acid group-containing monomers can selectively include other water-soluble polymers. In some specific examples, water-soluble polymers can include but not limited to partially saponified or completely saponified polyvinyl alcohol, polyethylene glycol, polyacrylic acid, polyacrylamide, starch and/or starch derivatives (e.g., methyl cellulose, methyl cellulose acrylate and/or ethyl cellulose). Preferably, the water-soluble polymer can be starch and/or partially or fully saponified polyvinyl alcohol. In some embodiments, based on the usage amount of the aqueous solution of acid group-containing monomers as 100 weight percent, a usage amount of the water-soluble polymers can be 0 weight percent to 20 weight percent, preferably 0 weight percent to 10 weight percent, and more preferably 0 weight percent to 5 weight percent, for example. If the usage amount of the water-soluble polymers is within this range, the production cost of the superabsorbent polymer can be reduced, but the absorption properties of the superabsorbent polymer will not be affected, thereby being able to meet the requirements of the application.

As the free radical polymerization step is performed, the crosslinking agent subjects the formed cross-linking core structure to have a suitable crosslinking degree, thereby enhancing the processability of the obtained superabsorbent polymer. In some embodiments, the crosslinking agent can include but not limited to a compound having at least two unsaturated double bond groups, a compound having at least two epoxy groups, other suitable crosslinking agents, or a combination thereof.

In some specific examples, the at least two unsaturated double bond groups can include but not limited to N,N'-bis(2-propenyl)amine, N,N'-methylenebisacrylamide, N,N'-methylenedimethacrylamide, propylene acrylate, ethylene glycol diacrylate, polyethylene glycol diacrylate, ethylene glycol dimethacrylate, polyethylene glycol dimethyl acrylates, glycerin triacrylate, glycerin trimethacrylate, glycerol added ethylene oxide triacrylate, glycerol added ethylene oxide trimethacrylate, trimethylolpropane added ethylene oxide triacrylate ester, trimethylolpropane added ethylene oxide trimethacrylate, trimethylolpropane trimethacrylate, trimethylolpropane triacrylate, N,N,N-tris(2-propenyl)amine, ethyleneglycol diacrylate, polyoxyethylene triacrylate glycerol esters, triethylene polyoxyethylene glycerol triacrylate, and/or dipropylene triethylene glycol esters.

In some specific examples, the compound having at least two unsaturated epoxy groups can include but be not limited to sorbitol polyglycidyl ether, polyglycerol polyglycidyl ether, ethylene glycol diglycidyl ether, diethylene glycol diglycidyl ether, polyethylene glycol diglycidyl ether and/or diglycerol polyglycidyl ether.

Based on the total solid content of the superabsorbent polymer as 100 weight percent, the usage amount of the crosslinking agent is 0.001 weight percent to 5 weight percent and preferably 0.010 weight percent to 3.000 weight percent. When the usage amount of the crosslinking agent is within the range, the cross-linking core structure formed by the free radical polymerization step can have better mechanical properties, thereby being helpful to be applied in mechanical processing, and the obtained superabsorbent polymer can still have better absorption properties.

As the free radical polymerization step is performed, the free radical decomposed from the polymerization initiator can induce a free radical polymerization of the acid group-containing monomer compositions, thereby obtaining the cross-linking core structure. The polymerization initiator of the present invention can include but not limited to a thermal decomposition initiator, a redox initiator, other suitable polymerization initiators, or any mixture of the aforementioned polymerization initiator. In some specific examples, the thermal decomposition initiator can include but not limited to hydrogen peroxide, di-tertiary butyl peroxide, amide peroxide, persulfate (e.g., ammonium salt, alkali metal salt or the like), and/or azo compound (e.g., 2,2'-azobis(2-amidinopropane) dihydrochloride, 2,2'-azobis(N,N-dimethyleneisobutyramide) dihydrochloride salt or the like). In some specific examples, the redox initiator can include but not limited to acidic sulfite, thiosulfate, ascorbic acid, ferrous salt and/or ammonium persulfate.

If the polymerization initiator includes both the thermal decomposition initiator and the redox initiator, the redox initiator can first decompose and generate the free radicals, thereby inducing a first-stage polymerization reaction of the free radical polymerization reaction. At the same time, the thermal decomposition initiator decomposes with the heat generated in the first-stage polymerization reaction and induces a second stage of the polymerization reaction, further enhancing the reactivity of the free radical polymerization.

Based on the weight of the salt of the acid group-containing monomers being neutralized as 100 weight percent, the usage amount of the polymerization initiator is 0.001 weight percent to 10 weight percent and preferably 0.1 weight percent to 5 weight percent. When the usage amount of the polymerization initiator is 0.001 weight percent to 10.000 weight percent, the free radical polymerization reaction has suitable reactivity and is easier to control, thereby preventing the formed cross-linking core structure from having an excessive degree of polymerization and therefore forming gel lumps.

The aforementioned free radical polymerization step can be performed in a conventional batch reaction vessel or on a conveyor belt reactor. In some embodiments, after the free radical polymerization, the obtained cross-linking core structure is selectively cut with a crusher to obtain small hydrogels.

The diameter of the small hydrogels can be smaller than or equal to 2.00 mm, e.g., 0.05 mm to 1.50 mm, or smaller than or equal to 1.0 mm to 1.50 mm, for example. If small hydrogels has a diameter in the aforementioned range, their surface areas are bigger to be helpful for the removal of more moisture and residual monomers in the subsequent drying step, thereby obtaining the superabsorbent polymer with better absorption properties. Moreover, if the small hydrogels have diameters in the aforementioned range, the amount of fine powders of the obtained cross-linking core structure powder can be controlled in a suitable range after being subjected to the subsequent drying step and pulverizing step. It is noted that the narrower the particle size distribution of the small hydrogels is, the easier the time control and temperature control are in the subsequent drying step, thereby enhancing physical properties of the obtained superabsorbent polymer.

In some embodiments, the drying step can be a heat-drying treatment, a freeze-drying treatment, or a vacuum-drying treatment. In some specific examples, the temperature of the heat-drying treatment can be 100°C to 250°C, or 100°C to 180°C, for example, to remove moisture in the small hydrogels effectively.

After the drying step, the pulverizing step can be selectively performed to obtain a powder of the cross-linking core structure. In some embodiments, after the pulverizing step, a screening treatment can be selectively performed on the cross-linking core structure, so as to narrow down the particle size distribution of the cross-linking core structure to 0.06 mm to 1.00 mm, e.g., 0.10 mm to 0.85 mm. As such, the obtained superabsorbent polymer has preferable physical properties, and the dust amount can be decreased.

The cross-linking core structure obtained in operation 110 is an insoluble hydrophilic polymer, and the interior of the cross-linking core structure has a uniform bridging structure. To improve the quality (e.g., absorption properties, colloid strength, anti-caking, absorption rate, and/or permeability) of the subsequent obtained superabsorbent polymer, the cross-linking core structure can be subjected to a surface-treating step with the surface-treating agent including the microcrystalline cellulose, as shown in operation 120, such that the surface of the crosslinked core structure is further bridged. In some embodiments, the time of the surface-treating step can be 0.5 hours to 1.5 hours, and the temperature can be 130°C to 180°C, for example, to achieve a better modification effect.

The aforementioned surface-treating agent is preferably to have multi-function groups that can react with acid groups. The conventional method of the surface-treating step can be: blending the cross-linking core structure with the surface-treating agent in an organic solvent (please refer to Japan patent publications numbers JP 1981-131608A, JP 1982-44627A, JP 1983-42602A, JP 1983-117222A); blending the surface-treating agent and the solution of the same in the cross-linking core structure with inorganic powders (please refer to Japan patent publications numbers JP 1985-163956A, JP 1985-255814A); performing a steaming treatment after adding the surface-treating agent (please refer to patent publication number JP 1989-113406A); mixing organic solvent, water and polyols as the surface-treating agent (please refer to Japan patent publication number JP 1989-292004A and the U.S. Pat. No. US 6,346,569B); blending organic solution, water, and ether compound (please refer to Japan patent publication number JP 1990-153903A). With the surface-treating step performed of the aforementioned conventional method, the obtained superabsorbent polymer can have an enhanced absorption rate and absorption against pressure. However, the retention capacity also decreases, thereby decreasing the performance of practical applications of the superabsorbent polymer.

The surface-treating agent used in the surface-treating step of the present invention includes but not limited to polyols, polyamines, compounds having at least two epoxy groups, alkylene carbonate, and/or other suitable compounds. In some specific examples, the polyols can include not limited to glycerol, ethylene glycol, diethylene glycol, triethylene glycol, polyethylene glycol, and/or propylene glycol. In some specific examples, the polyamines can include ethylenediamine, diethylenediamine, and/or triethylenediamine. In some specific examples, the compounds having at least two epoxy groups may include sorbitol polyglycidyl ether, polyglycerol polyglycidyl ether, ethylene glycol diglycidyl ether, diethylene glycol diglycidyl ether, polyethylene diol diglycidyl ether and/or diglycerol polyglycidyl ether. In some specific examples, the alkylene carbonate can include ethylene glycol carbonate, 4-methyl-1,3-dioxolan-2-one, 4,5-Dimethyl-1,3-dioxolan-2-one, 4,4-Dimethyl-1,3-dioxolan-2-one, 4-ethyl-1,3-dioxolan-2-one, 1,3-dioxan-2-one, 4,6-dimethyl-1,3-dioxane-2-keto and/or 1,3-dioxepan-2-one.

The aforementioned surface-treating agent can be used alone or a combination of two or more. In some specific examples, the volume ratio of the surface-treating agent can include but not limited to ethylene glycol, 1,4-butanediol (manufactured by Formosa Plastics Corporation), and methanol with a volume ratio of 1:1-(0.5-0.8). Based on the total solid content of the superabsorbent polymer as 100 weight percent, the usage amount of the surface-treating agent is 0.001 weight percent to 10 weight percent and preferably 0.005 weight percent to 5 weight percent. If the usage amount of the surface-treating agent is in the aforementioned range, the aforementioned crosslinking effect can be enhanced effectively, and the physical properties of the superabsorbent polymer such as absorption properties can still remain excellent.

As the surface-treating step performs, the surface-treating agent can be added therein directly; alternatively, the surface-treating agent can be prepared as a surface-treating agent aqueous solution with or without a hydrophilic organic solvent, followed by blending with the cross-linking core structure for performing the surface-treating step, depending on the types of the surface crosslinking agent. There are no specific limitations on the hydrophilic organic solvent as long as it can form a solution. In some embodiments, the hydrophilic organic solvent can be methanol, ethanol, propanol, isobutanol, acetone, methyl ether, and/or ethyl ether. In some specific examples, the hydrophilic organic solvent can be methanol and/or ethanol (please refer to the U.S. Pat. No. US 6,849,665B).

As shown in operation 120, the surface-treating agent includes microcrystalline cellulose. In some embodiments, the surface-treating step is performed after the microcrystalline cellulose is mixed with the surface-treating agent. In other embodiments, the microcrystalline cellulose is blended with the surface-treating agent and the cross-linking core structure directly. After the surface-treating step, the surface-treating agent including the microcrystalline cellulose forms a shell layer on the outer surface of the cross-linking core structure, thereby obtaining the superabsorbent polymer of the present invention, in which the shell layer encloses the cross-linking core structure completely, and the shell layer includes microcrystalline cellulose.

The microcrystalline cellulose can be obtained by subjecting cellulose with mineral acid (e.g., sulfuric acid, phosphoric acid, or hydrochloric acid) to the hydrolyzed amorphous regions of the cellulose so that the obtained microcrystalline cellulose has high crystallinity. The size of the microcrystalline cellulose depends on the source of cellulose, the specific condition of hydrolysis, the ionic strength, and the diffusion-controlled nature of acid hydrolysis. Generally, microcrystalline cellulose can have a width of 5 nm to 20 nm, a length of 100 nm to 500 nm, and an aspect ratio of 10 to 100, for example.

In some embodiments, the degree of polymerization of the microcrystalline cellulose can be smaller than 350, or 200 to 300, for example. In some embodiments, the microcrystalline cellulose is in powder, and the particle size can be 45 μm to 75 μm, for example. If the degree of polymerization and the particle size of the microcrystalline cellulose powder are in the aforementioned ranges, the absorption rate and permeability of the obtained superabsorbent polymer can be effectively enhanced.

In some embodiments, the bulk density of the microcrystalline cellulose can be 0.25 to 0.35, for example. In some embodiments, the moisture content of the microcrystalline cellulose can be smaller than or equal to 10%, e.g., bigger than 0% and smaller than or equal to 7%, or bigger than 0% and smaller than or equal to 4%, for example, so that the microcrystalline cellulose has better hydrophilicity. If the size of the microcrystalline cellulose is in the aforementioned range, the absorption rate and permeability of the superabsorbent polymer can be effectively enhanced, thereby meeting the requirements.

In some embodiments, the microcrystalline cellulose can be commercial products such as DUPONT AVICEL® PH-101, PH-301, PH-103, PH-113I; Hozhou Zhanwang pharmaceutical ZW-105, ZW-112, ZW-301; Asahi KASEI PH-101, and PH-102.

Based on the weight of the cross-linking core structure of the superabsorbent polymer as 100 weight percent, the usage amount of the microcrystalline cellulose is 0.1 weight percent to 5.0 weight percent, e.g., 0.2 weight percent to 5.0 weight percent, so that the obtained superabsorbent polymer can have better absorption rate and permeability, and defects such as gel blocking can be improved.

The microcrystalline cellulose of the present invention can bind to the surface of the cross-linking core structure having high absorption properties subjected to the surface-treating step so that the obtained superabsorbent polymer have excellent absorption rate and permeability as well as good absorption properties at the same time. Accordingly, after the formed superabsorbent polymer absorbs a large amount of moisture, defects such as gel blocking will not be generated, and thus the superabsorbent polymer has good absorption properties. In the present invention, the microcrystalline cellulose that meets the criteria of the present invention can be used to make a superabsorbent polymer with excellent absorption rate and permeability that can be applied in hygiene products of different kinds, such as water absorbents for use in agriculture and food preservation.

The obtained superabsorbent polymer of the present invention and the absorbent products made by applying the superabsorbent polymer can be used to make hygiene products, e.g., diapers, especially Fluffless diapers (using a large amount of the superabsorbent polymer) and adult diapers. When the conventional superabsorbent polymer is applied in diapers, the diapers cannot have excellent absorption rate and permeability as well as good absorption properties at the same time. However, the superabsorbent polymer of the present invention can effectively solve this problem.

The method of making a superabsorbent polymer and the obtained superabsorbent polymer of the present invention can enhance the absorption rate and permeability without reducing the absorption properties of the superabsorbent polymer.

Synthetic Example 1

A water solution of acrylic acid was obtained by blending 540 g of acrylic acid and 583.2 g of water. Next, 437.5 g of sodium hydroxide aqueous solution with a concentration of 48 weight percent was slowly dropped into the water solution of acrylic acid to perform a neutralizing step. The dropping ratio of sodium hydroxide and the acrylic acid was 0.85 to 0.95, the time of the neutralizing step was 2 hours, and during the neutralizing step, the temperature of the water solution of acrylic acid was kept at 15°C to 40°C. Moreover, the monomer concentration of the water solution of acrylic acid was 42 weight percent, and in the water solution of acrylic acid, 70 mole percent of the acrylic acid was neutralized to sodium acrylate.

Then, 0.9 g of N,N'-methylenebisacrylamide was added into the water solution of acrylic acid, and the temperature of the water solution of acrylic acid was kept at 20°C. Next, 0.3 g of hydrogen peroxide, 3.6 g of sodium bisulfite, and 3.6 g of ammonium persulfate starter were added to the water solution of acrylic acid, so as to perform a free radical polymerization step, thereby obtaining a hydrogel. The hydrogel was chopped with a cutting mill to obtain small hydrogels with particle sizes smaller than or equal to 2.0 mm. Thereafter, a drying step was performed on the small hydrogels at 130°C for 2 hours, followed by screening with a mesh of 0.1 mm to 0.85 mm to obtain a powder.

Preparation Example 1

The powder of Synthetic Example 1 was further subjected to a surface-treating step with a surface-treating agent, so as to obtain a superabsorbent polymer of Preparation Example 1. The surface-treating step was performed at 150°C for 1 hour. The surface-treating agent included ethylene glycol, 1,4-butanediol (manufactured by Formosa Plastics Corporation), and methanol with a volume ratio of 1:1:0.5, and the weight ratio of the powder of Synthetic Example 1 and the surface-treating agent was 200:5.

Synthetic Example 2

The method of making a powder of Synthetic Example 2 was the same as that of Synthetic Example 1, and the difference was that the method of making the powder of Synthetic Example 2 used 1.3 g of polyethylene glycol diacrylate with a molecular weight of 538 Da instead of N,N'-methylenebisacrylamide.

Preparation Example 2

The method of making the superabsorbent polymer of Preparation Example 2 was the same as that of Preparation Example 1. The difference was that the method of making Preparation Example 2 replaced the powder of Synthetic Example 1 with the powder of Synthetic Example 2.

Embodiment 1

The method of making a superabsorbent polymer of Embodiment 1 was the same as that of Preparation Example 1. The difference was that the surface-treating agent used in the method of Embodiment 1 included microcrystalline cellulose. Based on the weight of the powder of Preparation Example 1 as 100 weight percent, the usage amount of the microcrystalline cellulose was 0.5 weight percent. Moreover, the microcrystalline cellulose had a particle size of 20 $\mu$m, a degree of polymerization of 320, a moisture content of 6%, and a bulk density of 0.3.

Embodiments 2 to 6

The methods of making superabsorbent polymers of Embodiments 2 to 6 were the same as that of Embodiment 1, the differences were that the microcrystalline cellulose of Embodiment 2 had a particle size of 50 $\mu$m; the microcrystalline cellulose of Embodiment 3 had a particle size of 100 $\mu$m; the microcrystalline cellulose of Embodiment 4 had a particle size of 50 $\mu$m and a degree of polymerization of 230; the microcrystalline cellulose of Embodiment 5 had a particle size of 50 $\mu$m, a degree of polymerization of 230, and a moisture content of 3%; the microcrystalline cellulose of Embodiment 6 had a particle size of 50 $\mu$m, a degree of polymerization of 230, a moisture content of 3%, and a usage amount of 1.0 weight percent.

Embodiment 7

The method of making the superabsorbent polymer of Embodiment 7 was the same as that of Preparation Example 2. The difference was that the method to make Embodiment 7 used the surface-treating agent including microcrystalline cellulose, and the usage amount, the degree of polymerization, the particle size, the moisture content, and the bulk density were the same as that of the microcrystalline cellulose used in Embodiment 1.

Embodiments 8 to 12

The methods of making the superabsorbent polymers of Embodiments 8 to 12 were the same as that of Embodiment 7, the differences were that the microcrystalline cellulose of Embodiment 8 had a particle size of 50 $\mu$m; the microcrystalline cellulose of Embodiment 9 had a particle size of 100 $\mu$m; the microcrystalline cellulose of Embodiment 10 had a particle size of 50 $\mu$m, and the degree of polymerization was 230; the microcrystalline cellulose of Embodiment 11 had a particle size of 50 $\mu$m, a degree of polymerization was 230, and a moisture content of 3.0%; the microcrystalline cellulose of Embodiment 12 had a particle size of 50 $\mu$m, a degree of polymerization was 230, a moisture content of 3.0%, and a usage amount of 1.0 weight percent.

Comparative Embodiments 1 to 2

The methods of making the superabsorbent polymers of Comparative Embodiments 1 to 2 were the same as Embodiment 1. The differences were that the surface-treating agent of Comparative Embodiment 1 included lint cellulose with a degree of polymerization of 500 to 5000 instead of microcrystalline cellulose, and the surface-treating agent of Comparative Embodiment 2 included bleached Kraft pulp with a degree of polymerization of 1000 to 1500 instead of microcrystalline cellulose.

Comparative Embodiment 3

The methods of making superabsorbent polymer of Comparative Embodiment 3 were the same as that of Embodiment 1. The differences were that when the free radical polymerization step was performed, 100 ppm of phenol was added into the water solution of acrylic acid at first, followed by the addition of hydrogen peroxide, sodium bisulfite, and ammonium persulfate.

Comparative Embodiment 4

The method of making superabsorbent polymer of Comparative Embodiment 4 was the same as that of Comparative Embodiment 3. The differences were that the surface-treating agent of Comparative Embodiment 4 further included 0.2 g of silicon dioxide (manufacturer: Evonik; Cat. No.: A200).

Evaluation method and result

In the following evaluation methods, unless otherwise specified, the evaluation methods were performed at room temperature (e.g. 21° C. to 25° C.) and in an environment with relative air humidity of 35% to 55%, and the superabsorbent polymer were uniformly mixed before being evaluated.

Centrifuge Retention Capacity

The centrifuge retention capacity (CRC) was evaluated according to the measurement method of ERT 441.3(10) regulated by European Disposables and Nonwovens Association (EDANA).

Absorption Against Pressure (AAP)

The absorption against pressure (AAP) was evaluated according to the measurement method of ERT 442.3(10) regulated by EDANA, and was briefly described below: the initial weight of the superabsorbent polymer was measured, and then pressure of 4.9 kPa was exerted on the superabsorbent polymer, and weight of the superabsorbent polymer was measured after the superabsorbent polymer was merged in 0.9 weight percent of sodium chloride aqueous solution for 60 minutes. The absorption against pressure could be obtained by the weight after merging divided by the initial weight.

T20

T20 was measured by the dynamic effective permeability and absorption kinetics testing method (abbreviated as K(t) testing method in the following), and T20 represented the time needed for a superabsorbent polymer to absorb 20 g/g of water from the zero second as contacting a flow.

K(t) testing method was modified. Please refer to China patent publication number CN 103619291A for the modified K(t) testing method, and the modified K(t) testing method could be performed by a Time Dependent Permeability Tester (manufacture: BRAUN GmbH; Equipment No.: 03-080578) and was described briefly below: the dry superabsorbent polymer was spread on a mesh at the bottom of a measuring cylinder, and a given pressure was exerted to the superabsorbent polymer from the top, followed by a flow of saline from the top of the superabsorbent polymer to the bottom, so that the saline could maintain a hydrostatic head of 5 cm on the mesh at the bottom of the measuring cylinder. The thickness of the sample was measures every given time with a laser caliper gauge to determine the thickness change of the sample. The uptake of the superabsorbent polymer at the $t_i$ second ($U(t_i)$) could be obtained by formula (1).

$$U(t_i) = \frac{(A \times d_i - V_s)\rho}{m} \qquad (1)$$

In formula (1), A represented the cross-sectional area of the superabsorbent polymer on the mesh, di represented the difference of the thickness of the superabsorbent polymer at $t_i$ second and the thickness of the dry superabsorbent polymer; $V_s$ represented the space that the dry superabsorbent polymer substantially occupied, i.e., the volume of the superabsorbent polymer excluding the possible pores or gaps; $\rho$ represented the density of saline, and m represented the mass of the dry superabsorbent polymer.

Urine permeability measurement (UPM)

The specific measuring method for UPM could refer to the method described by WIPO patent publication number WO 2012/174026A, and would be described briefly below: the superabsorbent polymer was spread on the mesh at the bottom of the measuring cylinder. Then, the measuring cylinder was moved to the container containing a testing solution. As the mesh at the bottom of the measuring cylinder contacted the surface of the solution, the superabsorbent polymer on the mesh absorbed the testing solution for 60 minutes. Next, the testing solution flowed from the top of the superabsorbent polymer to the bottom to form and maintain a hydrostatic head of 5 cm. The volume of the testing solution flowing through the superabsorbent polymer was gathered for a given time at the bottom of the measuring cylinder, and the value of UPM was calculated according to formula (2).

$$Q = \frac{F_g \times L_0}{\rho \times A \times \Delta P} \qquad (2)$$

In formula (2), $F_g$ represented flow rate, which could be calculated by the slope of the regression line in the chart of flow to time (unit: g/second); $L_0$ represented the thickness of the dry superabsorbent polymer (unit: cm); $\rho$ represented the density of the testing solution (unit: $g/cm^3$); A represented the cross-sectional area of the superabsorbent polymer (unit: $cm^2$); $\Delta P$ represented the hydrostatic pressure (unit: $dyne/cm^2$); and Q represented UPM (unit: $cm^3 \cdot second/cm^2$).

Free-Swell Gel Bed Permeability (FSGBP) measuring method

By FSGBP measuring method, the gel bed permeability (GBP) under a swelling pressure of 0 psi could be obtained. GBP represented the liquid permeability of the superabsorbent polymer at a condition of free swelling. The term "free swelling" represented that the superabsorbent polymer was allowed to swell without a swelling restraint loading in a testing solution. The unit of GBP was "Darcy" which is a centimeter-gram-second (CGS) system unit of permeability. 1 Darcy was defined as the permeability of solid that in 1 second, a 1 $cm^3$ of fluid having a viscosity of 1 centipoise (cP) could pass through a section of the solid where the thickness was 1 cm and the cross-sectional area was 1 $cm^2$ as the difference of pressure between the two sides of the solid was 1 atm. 1 Darcy equaled to $0.98692 \times 10^{-8}$ $cm^2$. The FSGBP measuring method could refer to the method described by U.S. Pat. No. US 8,822,582B, and would not be elaborate hereafter.
The parameters of microcrystalline cellulose (including particle size, degree of polymerization, bulk density, and the use amount) used to make the samples of superabsorbent polymers of Preparation Examples 1 to 2, Embodiments 1 to 12, Comparative Embodiments 1 to 4 as well as the results (including centrifuge retention capacity, absorption against pressure, T20, GBP, and UPM) evaluated by the aforementioned methods were recorded in Table 1.

## Table 1

EP 4 659 769 A1

| Example | | Preparation Examples | | Embodiment | | | | | | | | | | | | Comparative Embodiment | | | |
|---|---|---|---|---|---|---|---|---|---|---|---|---|---|---|---|---|---|---|---|
| | | 1 | 2 | 1 | 2 | 3 | 4 | 5 | 6 | 7 | 8 | 9 | 10 | 11 | 12 | 1 | 2 | 3 | 4 |
| Use amount of SAP (g) | Synthetic Example 1 | 100 | 0 | 100 | 100 | 100 | 100 | 100 | 100 | 0 | 0 | 0 | 0 | 0 | 0 | 100 | 100 | 100 | 100 |
| | Synthetic Example 2 | 0 | 100 | 0 | 0 | 0 | 0 | 0 | 0 | 100 | 100 | 100 | 100 | 100 | 100 | 0 | 0 | 0 | 0 |
| Microcrystalline Cellulose | Particle size (μm) | | | 20 | 50 | 100 | 50 | 50 | 50 | 20 | 50 | 100 | 50 | 50 | 50 | Lint cellulose | Bleached Kraft pulp | Phenol | Silicon dioxide |
| | Degree of polymerization | | | 320 | 320 | 320 | 230 | 230 | 230 | 320 | 320 | 320 | 230 | 230 | 230 | | | | |
| | Moisture amount (%) | | | 6 | 6 | 6 | 6 | 3 | 3 | 6 | 6 | 6 | 6 | 3 | 3 | | | | |
| | Bulk density (g/ml) | | | 0.3 | 0.3 | 0.3 | 0.3 | 0.3 | 0.3 | 0.3 | 0.3 | 0.3 | 0.3 | 0.3 | 0.3 | | | | |
| | Use amount (%) | | | 0.5 | 0.5 | 0.5 | 0.5 | 0.5 | 1 | 0.5 | 0.5 | 0.5 | 0.5 | 0.5 | 1 | 0.5 | 0.5 | 0.5 | 0.5 |
| Evaluated results | CRC (g/g) | 32.5 | 33.5 | 32.2 | 32.3 | 32.2 | 32.5 | 33.5 | 33.4 | 33.2 | 33.3 | 33.3 | 33.6 | 34.2 | 34.1 | 31 | 31.2 | 31.5 | 31.7 |
| | AAP (g/g) | 22.5 | 23.7 | 21.6 | 21.5 | 21.7 | 21.4 | 21.3 | 21.8 | 23.2 | 23.1 | 23.0 | 23.2 | 22.7 | 23.2 | 23 | 22.9 | 22.1 | 16.5 |
| | T20 (second) | 385 | 305 | 330 | 260 | 318 | 245 | 230 | 213 | 301 | 240 | 291 | 226 | 211 | 195 | 390 | 397 | 620 | 350 |
| | UPM ($10^{-7}$ cm$^3$ second/g) | 16.0 | 15.0 | 35.0 | 40.0 | 37.0 | 45.0 | 48.0 | 49.0 | 40.0 | 45.0 | 42.0 | 50.0 | 54.0 | 55.0 | 14.0 | 15.0 | 15.0 | 16.0 |
| | GBP ($10^{-9} \times$ cm$^2$) | 7.2 | 6.8 | 25.4 | 26.1 | 25.5 | 26.5 | 27.9 | 28.2 | 26.2 | 27.3 | 26.4 | 27.6 | 29.3 | 30.0 | 6.9 | 7.1 | 7.1 | 8.0 |

As shown in Table 1, the microcrystalline cellulose was added to the superabsorbent polymer of Embodiments 1 to 12 during the surface-treating step, so the values of T20, UPM, and GBP of Embodiments 1 to 6 were higher than that of Preparation Example 1, and the values of T20, UPM, and GBP of Embodiments 7 to 12 were higher than that of Preparation Example 2, indicating that the absorption rate and permeability of the superabsorbent polymer could be enhanced by the surface-treating step.

Moreover, lint cellulose and bleached Kraft pulp were added to the superabsorbent polymer of the Comparative Embodiments 1 and 2 during the surface-treating step, so the obtained superabsorbent polymers had lower values of CRC, and the T20 and UPM were not better than Preparation Example 1, indicating that lint cellulose and bleached Kraft pulp could not improve the absorption properties as well as absorption rate and permeability of the superabsorbent polymer.

Phenol and silicon dioxide were added into the superabsorbent polymers of Comparative Embodiments 3 and 4 during the surface-treating step, respectively, so the values of CRC and T20 of Comparative Embodiment 3 using phenol were not good, and the values of CRC and AAP of Comparative Embodiment 4 using silicon dioxide were not good, and both of them could not have better UPM and GBP compared to Preparation Example 1, indicating that the use of phenol and silicon dioxide could not improve the permeability of superabsorbent polymer, and even worsen the absorption properties.

From the aforementioned examples, it can be understood that in the superabsorbent polymer and the method of making the same of the present invention, the advantages are that microcrystalline cellulose is used as the surface-treating step performs, and the obtained superabsorbent polymer can not only have excellent absorption rate and permeability but also maintain good absorption properties, thereby being able to be applied in thinner absorbent products.

**Claims**

1.    A superabsorbent polymer, **characterized by** comprising:

   a cross-linking core structure, obtained by subjecting an aqueous solution of acid group-containing monomers, a crosslinking agent, and a polymerization initiator to a free radical polymerization step; and
   a shell layer, enclosing the cross-linking core structure, wherein the shell layer is formed by performing a surface-treating step on the cross-linking core structure with a surface-treating agent, the surface-treating agent comprising microcrystalline cellulose, and based on the cross-linking core structure as 100 weight percent, a usage amount of the microcrystalline cellulose is 0.1 weight percent to 5.0 weight percent.

2.    The superabsorbent polymer according to claim 1, wherein based on a total solid content of the superabsorbent polymer as 100 weight percent, a usage amount of the crosslinking agent is 0.001 weight percent to 5 weight percent.

3.    The superabsorbent polymer according to claims 1 or 2, wherein the polymerization initiator comprises a thermal decomposition initiator and/or a redox initiator.

4.    The superabsorbent polymer according to any one of claims 1 to 3, wherein based on a total solid content of the superabsorbent polymer as 100 weight percent, a usage amount of the surface-treating agent is 0.001 weight percent to 10 weight percent.

5.    The superabsorbent polymer according to any one of claims 1 to 4, wherein a particle size of the microcrystalline cellulose is 20 $\mu$m to 100 $\mu$m.

6.    The superabsorbent polymer according to any one of claims 1 to 5, wherein a bulk density of the microcrystalline cellulose is 0.25 to 0.35.

7.    The superabsorbent polymer according to any one of claims 1 to 6, wherein a moisture content of the microcrystalline cellulose is smaller than or equal to 7%.

8.    The superabsorbent polymer according to any one of claims 1 to 7, wherein a degree of polymerization of the microcrystalline cellulose is smaller than or equal to 350.

9.    A method of making a superabsorbent polymer, **characterized by** comprising:
   performing a free radical polymerization step on a superabsorbent polymer composition, so as to form a cross-linking core structure, wherein the superabsorbent polymer composition comprises:

a aqueous solution of acid group-containing monomers;

a crosslinking agent; and

a polymerization initiator, comprising a thermal decomposition initiator and/or a redox initiator; and performing a surface-treating step on the cross-linking core structure with a surface-treating agent, so as to form a shell layer on the cross-linking core structure, wherein the shell layer encloses the cross-linking core structure completely, the surface-treating agent comprises microcrystalline cellulose, and based on the cross-linking core structure of the superabsorbent polymer is 100 weight percent, an usage amount of the microcrystalline cellulose is 0.1 weight percent to 5.0 weight percent, thereby obtaining the superabsorbent polymer.

**10.** The method according to claim 9, wherein a particle size of the microcrystalline cellulose is 20 $\mu$m to 100 $\mu$m.

**11.** The method according to claims 9 or 10, wherein a bulk density of the microcrystalline cellulose is 0.25 to 0.35.

**12.** The method according to any one of claims 9 to 11, wherein a moisture content of the microcrystalline cellulose is smaller than or equal to 7%.

**13.** The method according to any one of claims 9 to 12, wherein a degree of polymerization of the microcrystalline cellulose is smaller than or equal to 350.

**14.** The method according to any one of claims 9 to 13, further comprising a neutralizing step performed on the aqueous solution of acid group-containing monomers before the free radical polymerization step.

**15.** The method according to any one of claims 9 to 14, further comprising performing a drying step and/or a pulverizing step on the cross-linking core structure before the surface-treating step, so as to obtained a powder of the cross-linking core structure.

**Amended claims in accordance with Rule 137(2) EPC.**

**1.** A superabsorbent polymer, **characterized by** comprising:

a cross-linking core structure, obtained by subjecting an aqueous solution of acid group-containing monomers, a crosslinking agent, and a polymerization initiator to a free radical polymerization step; and a shell layer, enclosing the cross-linking core structure, wherein the shell layer is formed by performing a surface-treating step on the cross-linking core structure with a surface-treating agent, the surface-treating agent comprising microcrystalline cellulose, and based on the cross-linking core structure as 100 weight percent, a usage amount of the microcrystalline cellulose is 0.1 weight percent to 5.0 weight percent.

**2.** The superabsorbent polymer according to claim 1, wherein based on a total solid content of the superabsorbent polymer as 100 weight percent, a usage amount of the crosslinking agent is 0.001 weight percent to 5 weight percent.

**3.** The superabsorbent polymer according to claims 1 or 2, wherein the polymerization initiator comprises a thermal decomposition initiator and/or a redox initiator.

**4.** The superabsorbent polymer according to any one of claims 1 to 3, wherein based on a total solid content of the superabsorbent polymer as 100 weight percent, a usage amount of the surface-treating agent is 0.001 weight percent to 10 weight percent.

**5.** The superabsorbent polymer according to any one of claims 1 to 4, wherein a bulk density of the microcrystalline cellulose is 0.25 to 0.35.

**6.** The superabsorbent polymer according to any one of claims 1 to 5, wherein a moisture content of the microcrystalline cellulose is smaller than or equal to 7%.

**7.** A method of making a superabsorbent polymer, **characterized by** comprising: performing a free radical polymerization step on a superabsorbent polymer composition, so as to form a cross-linking core structure, wherein the superabsorbent polymer composition comprises:

a aqueous solution of acid group-containing monomers;

a crosslinking agent; and

a polymerization initiator, comprising a thermal decomposition initiator and/or a redox initiator; and

performing a surface-treating step on the cross-linking core structure with a surface-treating agent, so as to form a shell layer on the cross-linking core structure, wherein the shell layer encloses the cross-linking core structure completely, the surface-treating agent comprises microcrystalline cellulose, and based on the cross-linking core structure of the superabsorbent polymer is 100 weight percent, an usage amount of the microcrystalline cellulose is 0.1 weight percent to 5.0 weight percent, thereby obtaining the superabsorbent polymer.

8. The method according to claim 7, wherein a bulk density of the microcrystalline cellulose is 0.25 to 0.35.

9. The method according to any one of claims 7 to 8, wherein a moisture content of the microcrystalline cellulose is smaller than or equal to 7%.

10. The method according to any one of claims 7 to 9, further comprising a neutralizing step performed on the aqueous solution of acid group-containing monomers before the free radical polymerization step.

11. The method according to any one of claims 7 to 10, further comprising performing a drying step and/or a pulverizing step on the cross-linking core structure before the surface-treating step, so as to obtained a powder of the cross-linking core structure.

100

| |
|---|
| Perform a free radical polymerization step |
⌐110

↓

| |
|---|
| Perform a surface-treating step with a surface-treating agent including microcrystalline cellulose, so as to obtain a superabsorbent polymer |
⌐120

FIG. 1

Europäisches
Patentamt
European
Patent Office
Office européen
des brevets

# EUROPEAN SEARCH REPORT

Application Number

EP 24 19 8481

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/150761 A1 (LANGE NANCY BIRBIGLIA [US] ET AL) 17 October 2002 (2002-10-17) * paragraph [0044] - paragraph [0044]; claims 47, 48, 66, 70, 89; tables 1, 3, 5 * | 1-15 | INV. A61L15/24 A61L15/34 A61L15/60 B01J20/24 C08J3/12 |
| X | WO 00/62922 A1 (KIMBERLY CLARK CO [US]) 26 October 2000 (2000-10-26) * claims 1-42; figures 1-8; examples 1-2 * | 1-15 | C08J3/24 B01J20/30 |
| X | WO 00/62825 A2 (KIMBERLY CLARK CO [US]) 26 October 2000 (2000-10-26) * claims 1-50; figures 1-8; examples 1-2, 5 * | 1-15 | |

TECHNICAL FIELDS
SEARCHED        (IPC)

C08J
A61L
B01J

The present search report has been drawn up for all claims

| Place of search | Date of completion of the search | Examiner |
|---|---|---|
| Munich | 13 February 2025 | Marsitzky, Dirk |

EPO FORM 1503 03.82 (P04C01)

## ANNEX TO THE EUROPEAN SEARCH REPORT
## ON EUROPEAN PATENT APPLICATION NO.

EP 24 19 8481

This annex lists the patent family members relating to the patent documents cited in the above-mentioned European search report.
The members are as contained in the European Patent Office EDP file on
The European Patent Office is in no way liable for these particulars which are merely given for the purpose of information.

13-02-2025

| Patent document cited in search report | Publication date | Patent family member(s) | | Publication date |
|---|---|---|---|---|
| US 2002150761 A1 | 17-10-2002 | AR | 037761 A1 | 01-12-2004 |
| | | AU | 2002352891 A1 | 24-07-2003 |
| | | EP | 1465940 A2 | 13-10-2004 |
| | | KR | 20040068189 A | 30-07-2004 |
| | | MX | PA04005294 A | 13-09-2004 |
| | | US | 2002150761 A1 | 17-10-2002 |
| | | WO | 03057764 A2 | 17-07-2003 |
| WO 0062922 A1 | 26-10-2000 | AU | 4356200 A | 02-11-2000 |
| | | BR | 0008576 A | 23-04-2002 |
| | | CN | 1352574 A | 05-06-2002 |
| | | DE | 10084261 B3 | 25-07-2013 |
| | | GB | 2364506 A | 30-01-2002 |
| | | KR | 20020006034 A | 18-01-2002 |
| | | MX | PA01010255 A | 10-09-2004 |
| | | US | 6376011 B1 | 23-04-2002 |
| | | WO | 0062922 A1 | 26-10-2000 |
| WO 0062825 A2 | 26-10-2000 | AR | 039044 A1 | 09-02-2005 |
| | | AR | 042370 A2 | 22-06-2005 |
| | | AR | 042371 A2 | 22-06-2005 |
| | | AT | E305314 T1 | 15-10-2005 |
| | | AU | 760265 B2 | 08-05-2003 |
| | | BR | 0009808 A | 08-01-2002 |
| | | CA | 2362314 A1 | 26-10-2000 |
| | | CN | 1348388 A | 08-05-2002 |
| | | CO | 5160300 A1 | 30-05-2002 |
| | | CZ | 20013564 A3 | 12-06-2002 |
| | | DE | 60022871 T2 | 04-05-2006 |
| | | EP | 1171171 A2 | 16-01-2002 |
| | | JP | 2003519245 A | 17-06-2003 |
| | | KR | 20020013850 A | 21-02-2002 |
| | | MX | PA01010256 A | 10-09-2004 |
| | | PE | 20001408 A1 | 13-12-2000 |
| | | PL | 364857 A1 | 27-12-2004 |
| | | TR | 200102986 T2 | 21-03-2002 |
| | | TW | I231761 B | 01-05-2005 |
| | | US | 6387495 B1 | 14-05-2002 |
| | | WO | 0062825 A2 | 26-10-2000 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10335768 B
- JP 2001523289 A
- JP 2509087 B
- CN 1747751 A
- WO 2004113452 A
- US 20170050170 A
- US 5985944 A
- JP 6532894 B
- US 20050245684 A
- US 7777093 B
- WO 2006082189 A
- JP 56131608 A
- JP 57044627 A
- JP 58042602 A
- JP 58117222 A
- JP 60163956 A
- JP 60255814 A
- JP 1113406 A
- JP 1292004 A
- US 6346569 B
- JP 2153903 A
- US 6849665 B
- CN 103619291 A
- WO 2012174026 A
- US 8822582 B